# EUROPEAN PATENT APPLICATION

(11) **EP 3 295 874 A1**
(43) Date of publication of application: **21.03.2018**
(21) Application number: 16792797.9
(22) Date of filing: 13.05.2016
(51) Int. Cl.: A61B 6/04, A61B 6/03, A61N 5/10

(54) **FIXTURE FITTING DEVICE**

(30) Priority: 13.05.2015 JP 2015098631
(71) Applicant: Fujidenolo Co., Ltd., Komaki-shi, Aichi 485-0053 (JP); Medipolis Medical Research Institute, Ibusuki-shi, Kagoshima 891-0304 (JP)
(72) Inventor: SAKURAGI, Akari, Komaki-shi Aichi 485-0053 (JP); TANAKA, Fumitoshi, Komaki-shi Aichi 485-0053 (JP); TAKEUCHI, Norihide, Komaki-shi Aichi 485-0053 (JP); MIYAZAKI, Hideki, Komaki-shi Aichi 485-0053 (JP); ARIMURA, Takeshi, Ibusuki-shi Kagoshima 891-0304 (JP); MATSUYAMA, Mitsugi, Ibusuki-shi Kagoshima 891-0304 (JP); OGINO, Takashi, Ibusuki-shi Kagoshima 891-0304 (JP); HISHIKAWA, Yoshio, Ibusuki-shi Kagoshima 891-0304 (JP)
(74) Representative: Kuhnen & Wacker
(86) International application number: PCT/JP2016/064348
(87) International publication number: WO 2016/182073

(57) **Abstract**

Provided is a fixture fitting device that improves precision with which a fixture for holding a portion of the body of a subject is fitted to the subject. The fixture fitting device is provided with: a moving mechanism that moves the fixture 12 in the z-axis direction with respect to the body of the subject 8; an angle adjustment mechanism that adjusts the angle of the fixture 12 about the z-axis with respect to the body of the subject 8; and a pressure sensor 62 that detects pressure generated between the fixture 12 and a portion of the body of the subject 8 after the fixture 12 is brought into contact with the portion of the body, and this allows positioning using the moving mechanism and the angle adjustment mechanism while checking the pressure generated between the fixture 12 and the portion of the body of the subject 8. This leads to preventing a deviation from a desired fitting state even when the fixture 12 is fitted to a portion that is easily deformed.

## Description

### TECHNICAL FIELD

The present invention relates to a fixing-tool mounting device mounting a fixing tool holding a subject's body part on the subject.

### BACKGROUND ART

For example, a device is known that fixes a body of a subject to a bed when X-ray imaging or radiation treatment is performed by a medical device. For example, a subject fixing device of a medical device described in Patent Document 1 is an example thereof. According to this technique, a top plate of bed for placing a subject thereon includes a bag body surrounding the subject, and the bag body is made up of a skin formed of a hard strong material and an expansion chamber formed of a soft elastic material inside thereof, and it is described that by injecting a gas or a liquid into the expansion chamber by a compressor, the subject can securely be fixed to the top plate of the bed in the state of being surrounded by the bag body.

### PRIOR ART DOCUMENT

### Patent Document

Patent Document 1: Japanese Laid-Open Patent Publication No. 2007-301217

### SUMMARY OF THE INVENTION

### Problem to Be Solved by the Invention

However, depending on a part of a subject's body to be treated, the part cannot necessarily be held sufficiently securely with the conventional technique in some cases. For example, considering treatment to the breast, the breast easily deforms due to gravity in accordance with a posture etc. of the subject. Therefore, when it is assumed that a fixing tool formed in advance according to a shape of a body surface on a subject's body part is mounted on the subject, the part such as the breast to be treated may deform and thereby cause a deviation from a desired mounting form. Thus, a fixing-tool mounting device improving the accuracy of mounting of a fixing tool holding a subject's body part on the subject has not been developed yet at present.

The present invention was conceived in view of the situations and it is therefore an object of the present invention to provide a fixing-tool mounting device improving the accuracy of mounting of a fixing tool holding a subject's body part on the subject. Solution to Problem

To achieve the above object, a first aspect of the present invention provides a fixing-tool mounting device mounting a fixing tool including a curved surface formed in accordance with a shape of a body surface on a body part of a subject onto the body part of the subject set to a predetermined posture angle, comprising: a moving mechanism moving the fixing tool in at least one axial direction relative to the body of the subject; an angle adjustment mechanism adjusting an angle of the fixing tool around at least one axis relative to the body of the subject; and a pressure detecting portion, after the fixing tool contacts with the body part of the subject, detecting a pressure generated between the fixing tool and the body part.

### Advantageous Effects of the Invention

In the first aspect of the present invention, the fixing-tool mounting device includes the movement mechanisms moving the fixing tool in at least one axial direction relative to the body of the subject, the angle adjustment mechanism adjusting the angle of the fixing tool around at least one axis relative to the body of the subject, and the pressure detecting portion, after the fixing tool contacts with the body part of the subject, detecting the pressure generated between the fixing tool and the body part, and therefore, by performing positioning of the fixing tool relative to the body part with the movement mechanism and the angle adjustment mechanism while checking the pressure generated between the fixing tool and the body part, a deviation from a desired mounting form can be restrained from occurring even when the fixing tool is mounted onto an easily deformable part. This enables the provision of the fixing-tool mounting device improving the accuracy when the fixing tool holding the body part of the subject is mounted on the subject.

A second aspect of the present invention provides the fixing-tool mounting device recited in the first aspect of the invention, wherein the body part of the subject is a breast of the subject, wherein the fixing-tool mounting device comprises a horizontal adjustment mechanism making an adjustment to horizontalize a reference part to which the movement mechanism and the angle adjustment mechanism are attached, wherein the movement mechanism moves the fixing tool from the vertically lower side toward the vertically upper side to the breast of the subject turned to a prone position, and wherein the angle adjustment mechanism adjusts an angle of the fixing tool with regard to the vertical direction. Consequently, in the fixing-tool mounting device mounting the fixing tool from vertically lower side onto the breast of the subject, the accuracy can be improved when the fixing tool is mounted on the breast of the subject.

A third aspect of the present invention provides the fixing-tool mounting device recited in the first or second aspect of the invention, comprising a laser radiation device emitting a laser in the one axial direction. Consequently, by performing the positioning while applying the laser to the body part of the subject, a deviation from a desired mounting form can be restrained from occurring even when the fixing tool is mounted onto an easily deformable part.

A fourth aspect of the present invention provides the fixing-tool mounting device recited in any one of the first to third aspects of the invention, comprising a second movement mechanism moving the fixing tool in two axial directions perpendicular to the one axial direction and orthogonal to each other relative to the body of the subject. Consequently, the relative positions can more accurately be adjusted between the fixing tool and the body part of the subject.

A fifth aspect of the present invention provides the fixing-tool mounting device recited in any one of the first to fourth aspects of the invention, comprising an imaging device attached in a predetermined positional relationship with the fixing tool to photograph the body part of the subject. Consequently, by performing the positioning while checking the images taken by the imaging device, a deviation from a desired mounting form can be restrained from occurring even when the fixing tool is mounted onto an easily deformable part.

### BRIEF DESCRIPTION OF DRAWINGS

Fig. 1 is a perspective view for explaining an example of a configuration of a fixing-tool mounting device that is a preferred embodiment of the present invention.
Fig. 2 is a plane view of the fixing-tool mounting device shown in Fig. 1.
Fig. 3 is a side view of the fixing-tool mounting device shown in Fig. 1.
Fig. 4 is a perspective view of a state in which a fixing tool is attached to a fixing-tool attachment base of the fixing-tool mounting device of Fig. 1.
Fig. 5 is a perspective view of a state in which the fixing tool is detached from the fixing-tool attachment base of the fixing-tool mounting device of Fig. 1.
Fig. 6 is a perspective view of a state in which connection is released between the fixing-tool attachment base of the fixing-tool mounting device of Fig. 1 and the fixing tool.
Fig. 7 is a front view for explaining an example of the horizontal adjustment mechanism included in the fixing-tool mounting device shown in Fig. 1.
Fig. 8 is a perspective view for explaining an example of a specific operation of the fixing-tool mounting device shown in Fig. 1.
Fig. 9 is a diagram of an example of a screen displayed on an information terminal device when the fixing-tool mounting device shown in Fig. 1 is operated.
Fig. 10 is a perspective view of a state in which the fixing tool is raised in the vertical direction by a movement mechanism in the operation of the fixing-tool mounting device shown in Fig. 1.
Fig. 11 is a block diagram illustrating the constituents included in the fixing-tool mounting device shown in Fig. 1.
Fig. 12 is a flowchart for explaining a main portion in an example of control provided by an electronic control device etc. included in the fixing-tool mounting device shown in Fig. 1.

### MODES FOR CARRYING OUT THE INVENTION

Preferred embodiments of the present invention will now be described in detail with reference to the drawings. The drawings used for the following description are not necessarily accurately drawn in terms of dimensional ratios etc. of portions. Constituent elements common to embodiments are denoted by the same reference numerals and will not be described.

### Embodiments

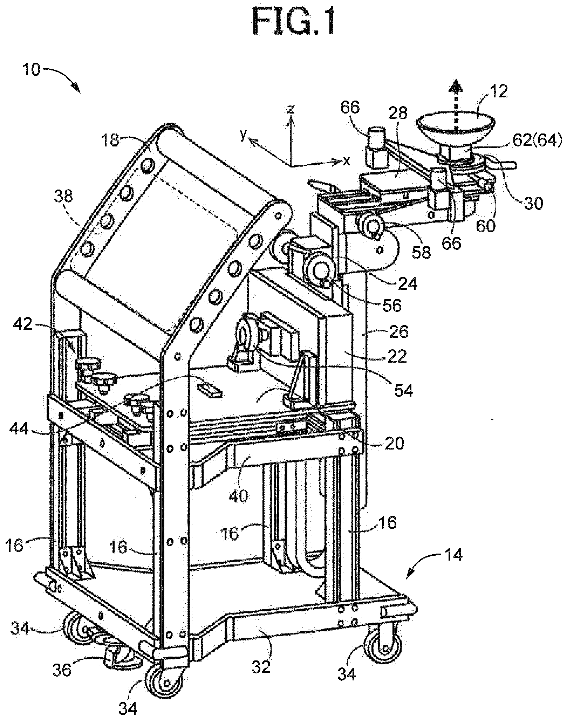

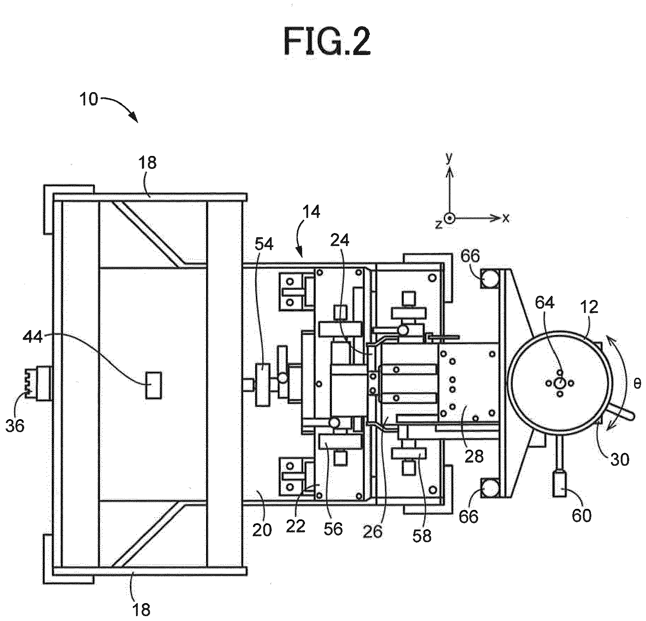

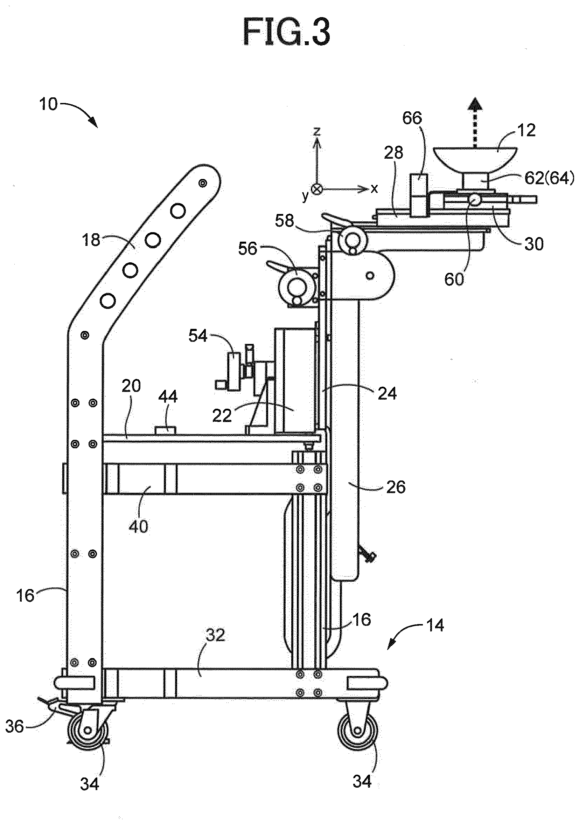

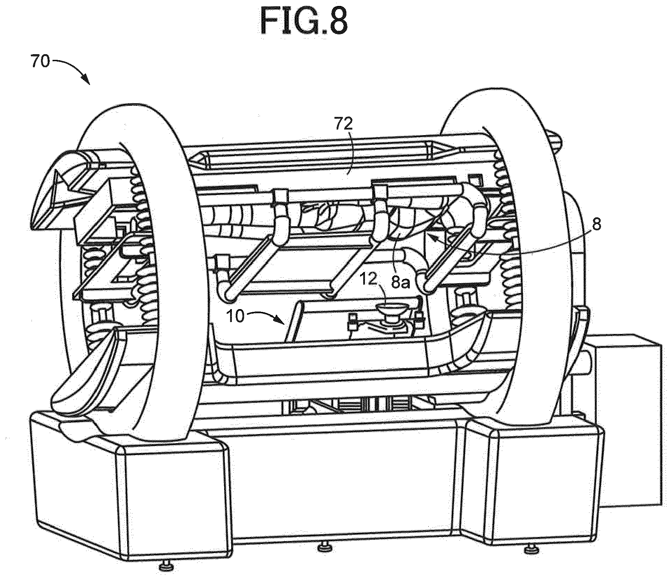

Fig. 1 is a perspective view for explaining an example of a configuration of a fixing-tool mounting device 10 that is a preferred embodiment of the present invention. Fig. 2 is a plane view of the fixing-tool mounting device 10 and Fig. 3 is a side view of the fixing-tool mounting device 10. In Figs. 1 to 3, the vertical direction is indicated by a z-axis, and the directions orthogonal to each other in the horizontal plane are indicated by an x-axis and a y-axis. The fixing-tool mounting device 10 of the present embodiment is, for example, a device mounting a fixing tool 12 on a body part of a subject 8 (see Fig. 8 etc. described later) in treatment such as particle beam treatment, X-ray treatment, γ-ray treatment, electron beam treatment, CT imaging, and MRI imaging by a medical device not shown. Therefore, the fixing-tool mounting device 10 is a tool to mount the fixing tool 12 holding a body part of the subject 8 during the treatment by the medical device on the body part of the subject 8 before the treatment.

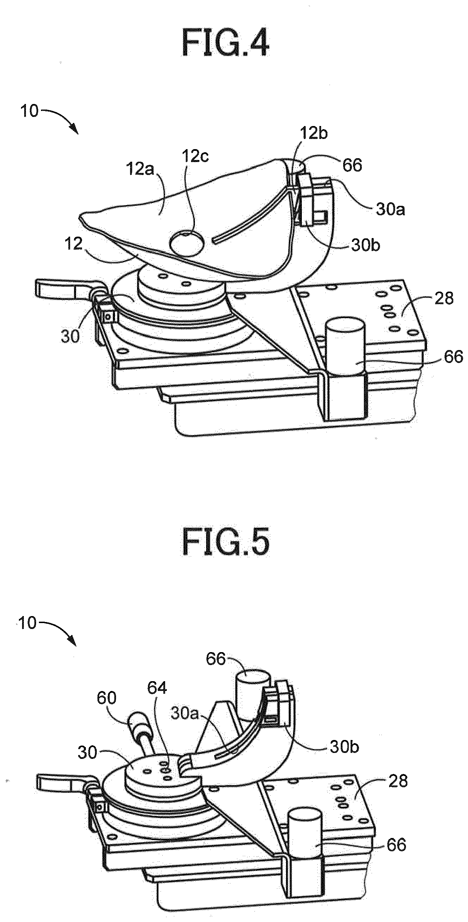

Fig. 4 is a perspective view of a state in which the fixing tool 12 is attached to the fixing-tool mounting device 10. The fixing tool 12 includes a curved surface 12a formed in accordance with a shape of a body surface on the body part of the subject 8. The fixing tool 12 includes a thin plate-shaped attaching portion 12b inserted into a slit 30a of a fixing-tool attachment base 30 when attached to the fixing-tool attachment base 30 described later. A main portion of configuration of the fixing tool 12 is made of a synthetic resin material. The fixing tool 12 is preferably formed of a synthetic resin material such as an acrylic resin, an ABS resin, and a polylactic resin. Alternatively, the fixing tool 12 may be formed of a fabric member made of fiber such as glass fiber, polyester fiber, or cotton impregnated with a curing agent and cured in accordance with the shape of the body surface on the body part.

The fixing tool 12 is preferably a breast fixing tool including the curved surface 12a as an inner surface formed into a cup shape in accordance with the shape of the body surface including at least one breast 8b of the subject 8 (see Fig. 8 etc.) along with an adhesive layer disposed on the curved surface 12a side thereof. The fixing tool 12 is formed into a shape partially or entirely covering the breast 8b including a papilla (nipple) of the subject 8. More preferably, the fixing tool 12 is formed into a shape partially or entirely covering a region including the breast 8b, the breastbone, the collarbone, and the ribs of the subject 8.

The fixing tool 12 includes the adhesive layer having adhesiveness to a skin surface of the subject 8 on the side of the curved surface 12a that is an inner surface formed in accordance with the shape of the body surface including the breast 8b. Therefore, when the fixing tool 12 is attached to the breast 8b of the subject 8, the adhesive layer having adhesiveness to the skin surface of the breast 8b is included on an entire or partial surface of a part of the fixing tool 12 coming into contact with the breast 8b. The adhesive layer is preferably made of a resin material having adhesiveness such as urethane gel or silicone gel. For the adhesive layer, an acrylic-, silicone-, urethane-, or rubber-based adhesive material may be used. For the adhesive layer, a synthetic resin material having adhesiveness such as acrylic-, silicone-, urethane-, styrene-based elastomer may be used.

Preferably, information related to the shape of the breast 8b of the subject 8 is acquired by a known three-dimensional scanner, for example, and the fixing tool 12 is individually produced for each of the subjects 8 based on the information. For example, the fixing tool 12 including the curved surface 12a corresponding to the shape of the breast 8b of the subject 8 is produced by a known 3D printer etc. based on the information. Alternatively, the fixing tool 12 matching the shape of the body surface on the body part of the subject 8 may be selected and used from multiple types of the fixing tools 12 having a shape corresponding to the body part of the subject 8 and formed into multiple sizes. Specifically, multiple types of the fixing tools 12 including the curved surfaces 12a formed in accordance with shapes of body surfaces on body parts of subjects 8 are prepared in advance correspondingly to respective multiple forms expected for the shape of the body surface on the body part. At the time of treatment of the subject 8, the fixing tool 12 matching the shape of the body surface on the body part of the subject 8 is selected and used from the multiple types of the fixing tools 12. For example, from the multiple types of the fixing tools 12 formed into sizes and shapes corresponding to the shape of the breast 8b (a type of so-called cup, a difference between top-bust and under-bust, etc.), the fixing tool 12 matching the shape of the body surface on the breast 8b of the subject 8 is appropriately selected and used.

The fixing-tool mounting device 10 of this embodiment mounts the fixing tool 12 configured as described above on the body part of the subject 8 set to a predetermined posture angle. As shown in Figs. 1 to 3, the fixing-tool mounting device 10 includes a cart 14, multiple (in this embodiment, four) supports 16, attaching portions 18, a plate member 20, a first member 22, a second member 24, a third member 26, a fourth member 28, and the fixing-tool attachment base 30. Preferably, the fixing-tool mounting device 10 includes a power-supply device such as a UPS (uninterruptible power supply) not shown made movable together with the fixing-tool mounting device 10 as the cart 14 moves.

The cart 14 includes a first frame 32, multiple (in this embodiment, four) casters 34 disposed on the lower side (vertically lower side) of the first frame 32, and a brake 36. The casters 34 are known caster devices including wheels with axle shafts of the wheels freely rotated by 360 ° in a plane parallel to a floor surface (the floor surface on which the fixing-tool mounting device 10 is disposed). The brake 36 is a known locking mechanism switching the states of preventing and permitting the rolling of the wheels of the casters 34. Preferably, the brake 36 switches the states of preventing and permitting the rolling of the wheels in the respective casters 34 at the same time (e.g., according to one operation).

The supports 16 are longitudinal (columnar) members and are disposed on the upper side (vertically upper side) of the first frame 32 such that the multiple supports 16 are substantially parallel to each other. The supports 16 are preferably fixed to the first frame 32 such that the longitudinal direction thereof substantially coincides with the vertical direction. The attaching portions 18 are parts disposed integrally with the two supports 16 on the side opposite to the side provided with constituents such as the fixing-tool attachment base 30 among the multiple supports 16. The attaching portions 18 are disposed on the upper side of the supports 16 in an extending manner and are inclined toward the center side of the fixing-tool mounting device 10 in the planar view shown in Fig. 2. An information terminal device 38 described later is attached to the attaching portions 18. The attaching portions 18 also serve as a gripping portion (handle portion) gripped by a person moving the fixing-tool mounting device 10 when moving the fixing-tool mounting device 10.

A second frame 40 is fixed to the supports 16. The second frame 40 is disposed on the upper side (vertically upper side) than the first frame 32 and substantially parallel to the first frame 32. The plate member 20 is attached to the second frame 40 via a horizontal adjustment mechanism 42. Therefore, the plate member 20 is configured such that the angle relative to the horizontal plane is adjusted by the horizontal adjustment mechanism 42 described below in detail. A level (level gauge) 44 is attached to the plate member 20 as a device for detecting whether the plate member 20 is horizontal. This level 44 is preferably a device enabling a user to detect whether the member is horizontal through visual observation, or may be a device such as a gyroscope sensor electrically detecting whether the plate member 20 is horizontal, for example. Although a configuration including the single level 44 is shown in Fig. 1 etc., multiple levels 44 may be included.

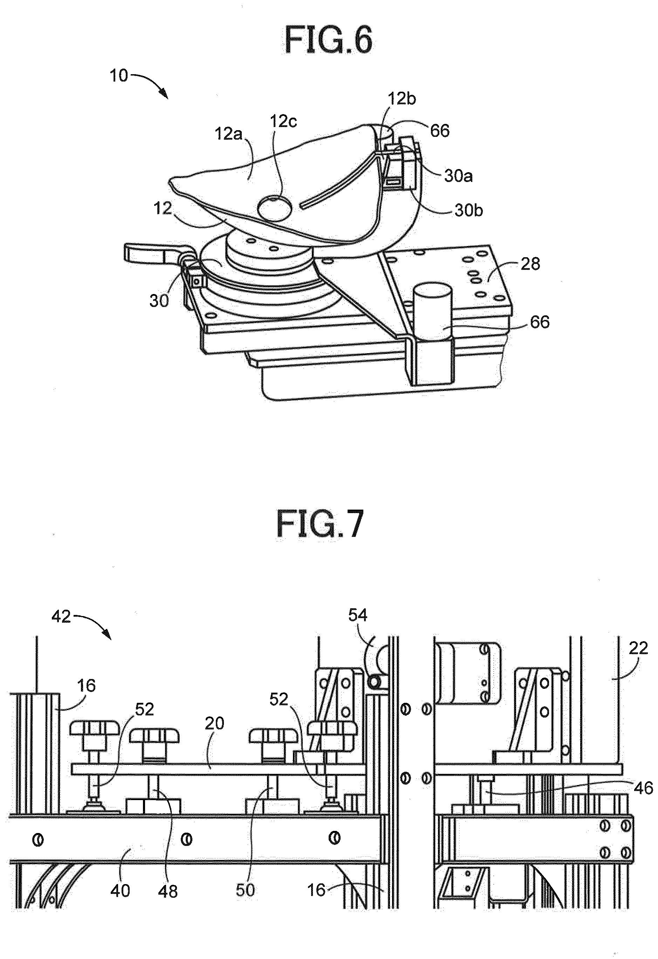

Fig. 7 is a front view for explaining an example of the horizontal adjustment mechanism 42 included in the fixing-tool mounting device 10. As shown in Fig. 7, the horizontal adjustment mechanism 42 adjusts the position of the plate member 20 relative to the second frame 40 at three points (three-point support) such that the plate member 20 becomes horizontal. Specifically, a first support shaft 46 is fixed to the plate member 20 and the second frame 40 and, in a portion corresponding to the first support shaft 46, a distance between the plate member 20 and the second frame 40 is made constant. A second support shaft 48 and a third support shaft 50 are made up of screws etc. respectively, each capable of adjusting a protrusion amount (an amount of protrusion thereof toward the second frame 40) from the plate member 20. By adjusting the distance between the plate member 20 and the second frame 40 in the respective portions corresponding to the second support shaft 48 and the third support shaft 50 while checking the level 44 through visual observation etc., the adjustment can be made so that the plate member 20 becomes horizontal. More preferably, the horizontal adjustment mechanism 42 includes multiple (in Fig. 7, two) auxiliary support shafts 52 supporting the plate member 20 with respect to the second frame 40 so as to restrain the plate member 20 from tilting due to the weight of the first member 22 etc. In the specifications of the fixing-tool mounting device 10, as described later, constituents such as the first member 22 serving as a mechanism adjusting the position and angle of the fixing tool 12 relative to the body of the subject 8 are all attached to the plate member 20, and therefore, in either case where the cart 14 is prevented from moving relative to the floor surface since the brake 36 is applied or where the cart 14 is permitted to move relative to the floor surface since the brake 36 is not applied, the adjustment can be made by the horizontal adjustment mechanism 42 such that the plate member 20 becomes horizontal.

The first member 22 is fixed to an upper surface (a surface on the upper side) of the plate member 20. The second member 24 is attached relatively movably in the y-axis direction with respect to the first member 22. For example, a Y-axis handle 54 is manually rotated to move the second member 24 relative to the first member 22 in the y-axis direction shown in Figs. 1 to 3 in this configuration. A movement stroke of the second member 24 in the y-axis direction relative to the first member 22 is about 200 mm, for example.

The third member 26 is attached relatively movably in the z-axis direction with respect to the second member 24. In other words, the third member 26 is attached relatively movably in the vertical direction. For example, a Z-axis handle 56 is manually rotated to move the third member 26 relative to the second member 24 in the z-axis direction, i.e., the vertical direction, shown in Figs. 1 to 3 in this configuration. A movement stroke in the z-axis direction of the third member 26 relative to the second member 24 is about 350 mm, for example. Preferably, a mechanism related to the relative movement of the third member 26 in the z-axis direction with respect to the second member 24 includes a balancer not shown. The balancer is a device maintaining the position of the third member 26 in the z-axis direction relative to the second member 24 while no external force is applied. For example, when the operation of the Z-axis handle 56 is stopped while the position of the third member 26 in the z-axis direction relative to the second member 24 is changed by operating the Z-axis handle 56, the balancer maintains the position of the third member 26 in the z-axis direction relative to the second member 24 at the time of the stop. Therefore, when the third member 26 is raised in the z-axis direction relative to the second member 24 by manually operating the Z-axis handle 56, a situation such as lowering of the third member 26 in the z-axis direction can be avoided at the moment of release of the Z-axis handle 56 from an operator's hand. Furthermore, a load required for the manual operation of the Z-axis handle 56 can advantageously be reduced.

The fourth member 28 is attached relatively movably in the x-axis direction with respect to the third member 26. For example, an X-axis handle 58 is manually rotated to move the fourth member 28 relative to the third member 26 in the x-axis direction shown in Figs. 1 to 3 in this configuration. A movement stroke of the fourth member 28 in the x-axis direction relative to the third member 26 is about 150 mm, for example.

The fixing-tool attachment base 30 is attached to the fourth member 28 such that the fixing-tool attachment base 30 moves relative to the fourth member 28 around at least one axis and an angle therebetween may be adjusted. For example, as shown in Fig. 2, the fixing-tool attachment base 30 is attached such that an angle θ around a rotation axis in the vertical direction (z-axis direction) may be adjusted. For example, the angle θ around the rotation axis of the fixing-tool attachment base 30 relative to the fourth member 28 can be changed by manually operating a 0-axis handle 60 in this configuration.

Figs. 4 to 6 are perspective views for explaining attachment of the fixing tool 12 to the fixing-tool attachment base 30. Fig. 5 shows the fixing-tool attachment base 30 when the fixing tool 12 is not attached. As shown in Fig. 5, the fixing-tool attachment base 30 is provided with the slit 30a having a width dimension allowing insertion of the attaching portion 12b of the fixing tool 12. The fixing-tool attachment base 30 includes a pressing member 30b moved between a locking position at which the width of the slit 30a is narrowed and a detaching position at which the width of the slit 30a is widened as compared to the locking position. Fig. 4 shows a state in which the pressing member 30b is at the locking position, and Figs. 5 and 6 show a state in which the pressing member 30b is at the detaching position. When the pressing member 30b is moved to the locking position with the attaching portion 12b of the fixing tool 12 inserted in the slit 30a of the fixing-tool attachment base 30, the attaching portion 12b is sandwiched between inner walls on both sides of the slit 30a, so that the fixing tool 12 is fixed to the fixing-tool attachment base 30 as shown in Fig. 4. The pressing member 30b is moved from this state to the detaching position as shown in Fig. 6 so as to achieve a state in which the fixing tool 12 is detachable from the fixing-tool attachment base 30.

While the fixing tool 12 is attached to the fixing-tool attachment base 30, as shown in Fig. 4, the position and angle of the fixing-tool attachment base 30 relative to the plate member 20 are adjusted by the Y-axis handle 54, the Z-axis handle 56, the X-axis handle 58, the 0-axis handle 60, etc. so as to adjust the position and angle of the fixing tool 12 relative to the body of the subject 8 set to a predetermined posture angle. Therefore, in this embodiment, the second member 24, the third member 26, and the Z-axis handle 56 correspond to a movement mechanism moving the fixing tool 12 in at least one axial direction (i.e., the z-axis direction) relative to the body of the subject 8. These correspond to the movement mechanism moving the fixing tool 12 from the vertically lower side toward the vertically upper side relative to the body of the subject 8. The first member 22, the second member 24, the third member 26, the fourth member 28, the Y-axis handle 54, and the X-axis handle 58 correspond to a second movement mechanism moving the fixing tool 12 in two axial directions perpendicular to the one axial direction and orthogonal to each other (i.e., the x-axis direction and the y-axis direction) relative to the body of the subject 8. The fourth member 28, the fixing-tool attachment base 30, and the 0-axis handle 60 correspond to an angle adjustment mechanism adjusting the angle of the fixing tool 12 around at least one axis relative to the body of the subject 8. These correspond to the angle adjustment mechanism adjusting the angle θ of the fixing tool 12 with regard to the vertical direction. The plate member 20 corresponds to a reference part to which the movement mechanism and the angle adjustment mechanism are attached.

The fixing-tool mounting device 10 includes a pressure sensor (load cell) 62 detecting a pressure generated, after the fixing tool 12 attached to the fixing-tool attachment base 30 comes into contact with a body part of the subject 8, between the fixing tool 12 and the body part. In this embodiment, the pressure sensor 62 corresponds to a pressure detecting portion. Preferably, the pressure sensor 62 detects pressures at multiple positions (e.g., three positions) between the fixing tool 12 and the body part of the subject 8. Preferably, the pressure sensor 62 is connected to the information terminal device 38 so that a signal detected by the pressure sensor 62 is supplied to the information terminal device 38.

The fixing-tool mounting device 10 includes a laser pointer 64 applying a laser in a uniaxial direction from the fixing-tool attachment base 30. In Figs. 1 and 3, the direction of the laser emitted from the laser pointer 64 is indicated by a thick dashed arrow. Preferably, the laser pointer 64 is a laser radiation device built into the fixing-tool attachment base 30 and emitting a laser of a predetermined color upward in a direction perpendicular to the fixing-tool attachment base 30, i.e., a substantially vertical direction (z-axis direction). Therefore, as shown in Figs. 4, 6, etc., the fixing tool 12 has a hole portion 12c formed at a position corresponding to the laser emitted from the laser pointer 64 in a state of being attached to the fixing-tool attachment base 30 and is configured not to block the laser emitted by the laser pointer 64. In this embodiment, the laser pointer 64 corresponds to a laser radiation device.

The fixing-tool mounting device 10 includes one or more (in this embodiment, two) cameras 66 attached in a predetermined positional relationship with the fixing tool 12 to photograph a body part of the subject 8. In this embodiment, the cameras 66 correspond to an imaging device. Although the configuration described in this embodiment includes the two cameras 66 so that a wide range image can be taken, the fixing-tool mounting device 10 may have a configuration including the single camera 66. The fixing-tool mounting device 10 may have a configuration including the three or more cameras 66. As shown in Figs. 1 to 3, the cameras 66 are disposed at fixed positions on the fixing-tool attachment base 30 so as to take an image on the upper side in the vertical direction. In other words, the cameras are disposed to take an image in the direction in which the fixing tool 12 is moved by the movement mechanism relative to the body of the subject 8, i.e., an image viewed from the vertically lower side toward the vertically upper side. The cameras 66 are preferably connected to the information terminal device 38 so that image information taken by the camera 66 is supplied to the information terminal device 38.

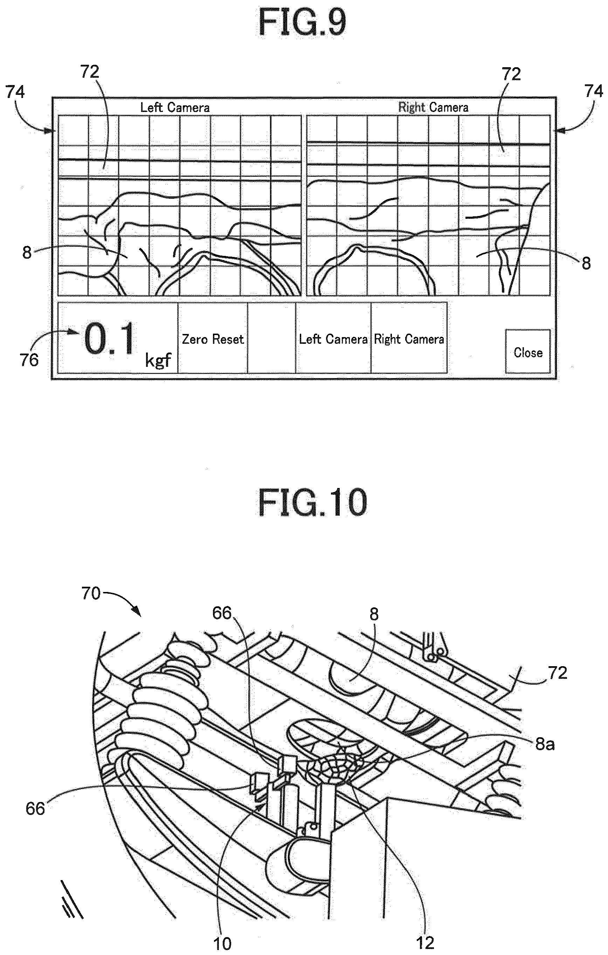

The information terminal device 38 is a known personal computer, a monitor, a tablet terminal, etc., and has at least an image display function. Preferably, the information terminal device 38 is attachable to and detachable from the attaching portion 18 of the fixing-tool mounting device 10. As shown in Fig. 9 described later, a numerical value indicative of the pressure generated between the fixing tool 12 and the body part and detected by the pressure sensor 62 is displayed on the information terminal device 38. An image including the body part of the subject 8 taken by the cameras 66 is displayed on the information terminal device 38. The information terminal device 38 may be a terminal functioning exclusively as an image display device or may be a terminal corresponding to a control device (controller) controlling the operation of the fixing-tool mounting device 10 as described later.

Fig. 8 is a perspective view for explaining an example of a specific operation of the fixing-tool mounting device 10 mounting the fixing tool 12 on the body part of the subject 8 set to a predetermined posture angle. The example shown in Fig. 8 represents a form in which the posture angle of the subject 8 is changed by a body position changing device 70. In the example shown in Fig. 8, the body of the subject 8 is fixed to a frame member 72 formed into a rectangular shape in a planar view. The body is fixed such that the head-foot direction of the subject 8 coincides with the longitudinal direction (long-side direction) of the frame member 72. The frame member 72 to which the subject 8 is fixed is attached to the body position changing device 70. The body position changing device 70 changes the angle of the frame member 72 relative to the vertical direction (the angle formed by a plane portion of the frame member 72 relative to the vertical direction). This results in a change in the posture angle of the subject 8 fixed to the frame member 72 relative to the vertical direction.

The fixing-tool mounting device 10 mounts the fixing tool 12 on the body part of the subject 8 set to a predetermined posture angle by the body position changing device 70. Preferably, the fixing tool 12 is mounted from the vertically lower side toward the vertically upper side onto the breast 8a of the subject 8 turned to the prone position (face-down) by the body position changing device 70. Specifically, while the angle of the frame member 72 relative to the vertical direction is set to a predetermined angle by the body position changing device 70, the fixing tool 12 is mounted from the vertically lower side toward the vertically upper side onto the breast 8a of the subject 8 fixed to the frame member 72.

A method of mounting the fixing tool 12 onto the body of the subject 8 by the fixing-tool mounting device 10 in the example shown in Fig. 8 will hereinafter specifically be described. First, the fixing-tool mounting device 10 having the fixing tool 12 attached thereto is moved to an arbitrary position relative to the body position changing device 70 to which the frame member 72 fixing the subject 8 is attached. The fixing-tool mounting device 10 is moved to a position where the fixing tool 12 is located substantially vertically under the breast 8a of the subject 8. The brake 36 is then operated into the state of preventing the rolling of the wheels of the casters 34.

The laser is then emitted from the laser pointer 64. The position of the fixing tool 12 in the horizontal plane is adjusted such that the laser emitted from the laser pointer 64 is applied to the nipple of the breast 8a of the subject 8. In particular, the Y-axis handle 54 is operated to adjust the position of the fixing tool 12 in the y-axis direction relative to the breast 8a of the subject 8, and the X-axis handle 58 is operated to adjust the position of the fixing tool 12 in the x-axis direction relative to the breast 8a of the subject 8.

The angle θ of the fixing tool 12 relative to the body of the subject 8 is then adjusted based on the image taken by the camera 66. Fig. 9 is a diagram of an example of a screen displayed on the information terminal device 38 when the fixing-tool mounting device 10 is operated. As shown in Fig. 9, when the fixing-tool mounting device 10 is operated, images 74 respectively taken by the two cameras 66 are displayed on the information terminal device 38. In the images 74, a grid (lattice) is compositely displayed as reference lines of directions in the horizontal plane. The angle θ of the fixing tool 12 is adjusted by operating the 0-axis handle 60 such that a straight line corresponding to the outer edge (edge) of the image of the frame member 72 included in the images 74 becomes parallel to the grid. Alternatively, the angle θ of the fixing tool 12 may be adjusted such that a straight line portion in the image of the body position changing device 70 included in the images 74 becomes parallel to the grid.

The fixing tool 12 is then moved from the vertically lower side toward the vertically upper side to the breast 8a of the subject 8 turned to the prone position by the body position changing device 70. In particular, the Z-axis handle 56 is operated to adjust the position of the fixing tool 12 in the z-axis direction, i.e., the vertical direction, relative to the breast 8a of the subject 8 as shown in Fig. 10. After the fixing tool 12 contacts with the breast 8a of the subject 8, as shown in Fig. 9, a value 76 indicative of the pressure detected by the pressure sensor 62 is displayed on the screen of the information terminal device 38. When the value 76 indicative of the pressure reaches a predetermined value, the raising of the fixing tool 12 in the z-axis direction is stopped. Consequently, the fixing tool 12 is affixed to the breast 8a at a necessary and sufficient pressure by the adhesive layer included on the curved surface 12a.

Next, the pressing member 30b of the fixing-tool attachment base 30 is moved to the detaching position as shown in Fig. 6 to release the connection between the fixing tool 12 and the fixing-tool attachment base 30. By operating the Z-axis handle 56, the fixing-tool attachment base 30 is then lowered to its lower end in the z-axis direction, i.e., in the vertical direction, and the fixing tool 12 is detached from the fixing-tool attachment base 30. As described above, the fixing tool 12 is mounted on the breast 8a of the subject 8. After the fixing tool 12 is mounted on the breast 8a of the subject 8, for example, the fixing tool 12 is fixed to the frame member 72. Consequently, the breast 8a of the subject 8 is fixed with respect to the frame member 72, and the subsequent treatment is performed in this state.

In the embodiment described above, the movement mechanism, the second movement mechanism, and the angle adjustment mechanism adjust the position and angle of the fixing tool 12 relative to the body of the subject 8 through the manual operations of the Y-axis handle 54, the Z-axis handle 56, the X-axis handle 58, the 0-axis handle 60, etc.; however, the fixing-tool mounting device 10 may use an electric actuator to adjust the position and angle of the fixing tool 12 relative to the body of the subject 8.

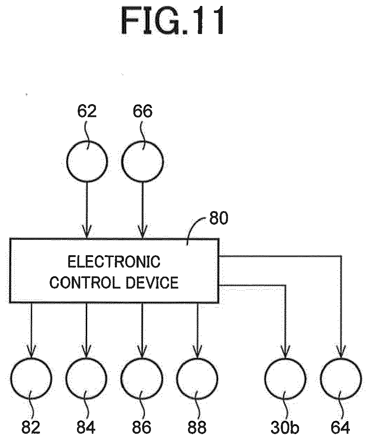

Fig. 11 is a block diagram illustrating the constituents included in the fixing-tool mounting device 10. As shown in Fig. 11, instead of or in addition to the Y-axis handle 54, the fixing-tool mounting device 10 may include a first electric actuator 82 generating a power for relatively moving the second member 24 with respect to the first member 22 in the y-axis direction shown in Figs. 1 to 3. Instead of or in addition to the Z-axis handle 56, the fixing-tool mounting device 10 may include a second electric actuator 84 generating a power for relatively moving the third member 26 with respect to the second member 24 in the z-axis direction shown in Figs. 1 to 3. Instead of or in addition to the X-axis handle 58, the fixing-tool mounting device 10 may include a third electric actuator 86 generating a power for relatively moving the fourth member 28 with respect to the third member 26 in the x-axis direction shown in Figs. 1 to 3. Instead of or in addition to the θ-axis handle 60, the fixing-tool mounting device 10 may include a fourth electric actuator 88 generating a power for changing the angle θ around the rotation axis of the fixing-tool attachment base 30 relative to the fourth member 28.

The fixing-tool mounting device 10 preferably includes an electronic control device 80 as a controller controlling the driving of the first electric actuator 82, the second electric actuator 84, the third electric actuator 86, the fourth electric actuator 88, etc. In such a form, the pressure sensor 62 is connected to the electronic control device 80 so that a signal detected by the pressure sensor 62 is supplied to the electronic control device 80. The laser pointer 64 is connected to the electronic control device 80 so that the laser is emitted from the laser pointer 64 according to a command from the electronic control device 80. The camera 66 is connected to the electronic control device 80 so that image information taken by the camera 66 is supplied to the electronic control device 80. The pressing member 30b of the fixing-tool attachment base 30 is configured to be switched between the locking position and the detaching position according to a command from the electronic control device 80. The information terminal device 38 may function as a controller controlling the driving of the constituents.

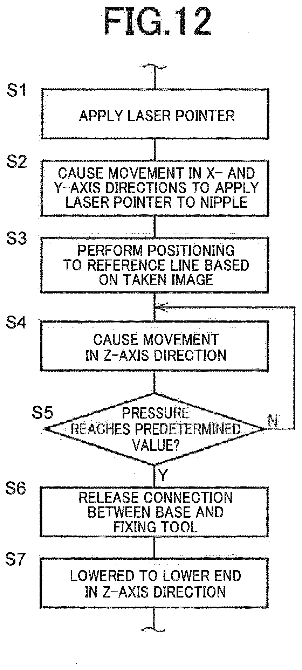

Fig. 12 is a flowchart for explaining a main portion in an example of control provided by the electronic control device 80 or the information terminal device 38 etc. included in the fixing-tool mounting device 10. First, at step (hereinafter, step is omitted) S1, the laser emission from the laser pointer 64 is started. At S2, the driving of the first electric actuator 82 and the third electric actuator 86 is controlled such that the laser emitted from the laser pointer 64 is applied to the nipple of the breast 8a of the subject 8 to adjust the position of the fixing tool 12 in the x-axis direction and the y-axis direction. At S3, the images 74 taken by the cameras 66 are analyzed, and the driving of the fourth electric actuator 88 is controlled such that a straight line corresponding to the outer edge (edge) of the image of the frame member 72 included in the images 74 becomes parallel to the grid serving as the reference lines to adjust the angle θ of the fixing tool 12. At S4, the driving of the second electric actuator 84 is controlled to raise the fixing tool 12 in the z-axis direction. At S5, it is determined whether the value indicative of the pressure detected by the pressure sensor 62 has reached a predetermined value. If the determination of S5 is negative, the process from S4 is executed again and, if the determination of S5 is affirmative, the pressing member 30b of the fixing-tool attachment base 30 is switched to the detaching position at S6. At S7, the driving of the second electric actuator 84 is controlled to lower the fixing tool 12 to the lower end in the z-axis direction, and this routine is then terminated.

In the control shown in Fig. 12, all the steps may not necessarily be executed. For example, S4 and S5 may be executed without executing the remaining steps. In particular, while the electronic control device 80 controls the driving of the second electric actuator 84 to implement the operation of raising the fixing tool 12 in the z-axis direction and stopping the raising when the pressure detected by the pressure sensor 62 reaches a predetermined value, the position adjustment of the fixing tool 12 in the horizontal plane, the adjustment of the angle θ, etc. may be implemented by manual operations.

In this embodiment, the fixing-tool mounting device 10 includes the second member 24, the third member 26, and the Z-axis handle 56 as the movement mechanisms moving the fixing tool 12 in at least one axial direction relative to the body of the subject 8, the fourth member 28, the fixing-tool attachment base 30, and the 0-axis handle 60 as the angle adjustment mechanism adjusting the angle of the fixing tool 12 around at least one axis relative to the body of the subject 8, and the pressure sensor 62 as the pressure detecting portion, after the fixing tool 12 contacts with the body part of the subject 8, detecting the pressure generated between the fixing tool 12 and the body part, and therefore, by performing positioning of the fixing tool 12 relative to the body part with the movement mechanism and the angle adjustment mechanism while checking the pressure generated between the fixing tool 12 and the body part, a deviation from a desired mounting form can be restrained from occurring even when the fixing tool 12 is mounted onto an easily deformable part. This enables the provision of the fixing-tool mounting device 10 improving the accuracy when the fixing tool 12 holding the body part of the subject 8 is mounted on the subject 8.

The body part of the subject 8 is the breast 8a of the subject 8; the fixing-tool mounting device 10 includes the horizontal adjustment mechanism 42 making an adjustment to horizontalize the plate member 20 serving as the reference part to which the movement mechanism and the angle adjustment mechanism are attached; the movement mechanism moves the fixing tool 12 from the vertically lower side toward the vertically upper side to the breast 8a of the subject 8 turned to the prone position; the angle adjustment mechanism adjusts the angle θ of the fixing tool 12 with regard to the vertical direction; and therefore, in the fixing-tool mounting device 10 mounting the fixing tool 12 from vertically lower side onto the breast 8a of the subject 8, the accuracy can be improved when the fixing tool 12 is mounted on the breast 8a of the subject 8.

The fixing-tool mounting device 10 includes the laser pointer 64 as a laser radiation device emitting a laser in the one axial direction, and therefore, by performing the positioning while applying the laser to the body part of the subject 8, a deviation from a desired mounting form can be restrained from occurring even when the fixing tool 12 is mounted onto an easily deformable part.

Since the fixing-tool mounting device 10 includes the first member 22, the second member 24, the third member 26, the fourth member 28, the Y-axis handle 54, and the X-axis handle 58 as the second movement mechanism moving the fixing tool 12 in two axial directions perpendicular to the one axial direction and orthogonal to each other relative to the body of the subject 8, the relative positions can more accurately be adjusted between the fixing tool 12 and the body part of the subject 8.

The fixing-tool mounting device 10 includes the cameras 66 as the imaging device attached in a predetermined positional relationship with the fixing tool 12 to photograph the body part of the subject 8, and therefore, by performing the positioning while checking the images 74 taken by the cameras 66, a deviation from a desired mounting form can be restrained from occurring even when the fixing tool 12 is mounted onto an easily deformable part.

Although the preferred embodiments of the present invention have been described in detail with reference to the drawings, the present invention is not limited to these embodiments and is implemented with other various modifications without departing from the spirit thereof.

### REFERENCE SIGNS LIST

8: Subject 8a: Breast (Body part) 10: Fixing-tool mounting device 12: Fixing tool 12a: Curved surface 20: Plate member (Reference part) 42: Horizontal adjustment mechanism 62: Pressure sensor (Pressure detecting portion) 64: Laser pointer (Laser radiation device) 66: Camera (Imaging device)

## Claims

1. A fixing-tool mounting device mounting a fixing tool including a curved surface formed in accordance with a shape of a body surface on a body part of a subject onto the body part of the subject set to a predetermined posture angle, comprising:
a moving mechanism moving the fixing tool in at least one axial direction relative to the body of the subject;
an angle adjustment mechanism adjusting an angle of the fixing tool around at least one axis relative to the body of the subject; and
a pressure detecting portion configured to, after the fixing tool contacts with the body part of the subject, detect a pressure generated between the fixing tool and the body part.

2. The fixing-tool mounting device according to claim 1, wherein
the body part of the subject is a breast of the subject, wherein
the fixing-tool mounting device comprises a horizontal adjustment mechanism making an adjustment to horizontalize a reference part to which the movement mechanism and the angle adjustment mechanism are attached, wherein
the movement mechanism moves the fixing tool from the vertically lower side toward the vertically upper side to the breast of the subject turned to a prone position, and wherein
the angle adjustment mechanism adjusts an angle of the fixing tool with regard to the vertical direction.

3. The fixing-tool mounting device according to claim 1 or 2, comprising a laser radiation device emitting a laser in the one axial direction.

4. The fixing-tool mounting device according to any one of claims 1 to 3, comprising
a second movement mechanism moving the fixing tool in two axial directions perpendicular to the one axial direction and orthogonal to each other relative to the body of the subject.

5. The fixing-tool mounting device according to any one of claims 1 to 4, comprising
an imaging device attached in a predetermined positional relationship with the fixing tool to photograph the body part of the subject.
